(19) Europäisches Patentamt
European Patent Office
Office européen des brevets

(11) Publication number: 0 095 394
B1

(12) # EUROPEAN PATENT SPECIFICATION

(45) Date of publication of patent specification: **10.12.86**

(51) Int. Cl.⁴: **C 07 F 9/65,** A 61 K 31/675

(21) Application number: **83400750.2**

(22) Date of filing: **15.04.83**

(54) **2-Oxo-1-(thiophosphonic or thiophosphinic)-azetidines.**

(30) Priority: **24.05.82 US 381260**

(43) Date of publication of application:
**30.11.83 Bulletin 83/48**

(45) Publication of the grant of the patent:
**10.12.86 Bulletin 86/50**

(84) Designated Contracting States:
**AT BE CH DE FR GB IT LI LU NL SE**

(56) References cited:
**EP-A-0 021 678**
**EP-A-0 061 765**

(73) Proprietor: **E.R. Squibb & Sons, Inc.**
**Lawrenceville-Princeton Road**
**Princeton, N.J. 08540 (US)**

(72) Inventor: **Zahler, Robert**
**Meadow Lane Apts. Meadow Road**
**Princeton New Jersey (US)**
Inventor: **Koster, William H.**
**R.D. No. 1 Welisewitz Road**
**Ringoes New Jersey (US)**

(74) Representative: **Maiffret, Bernard et al**
**Law Offices of William J. Rezac 49, avenue**
**Franklin D. Roosevelt**
**F-75008 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 095 394 B1

**Description**

EP 21678 discloses 1-sulfo-2-oxoazetidinone compounds as antibiotics.

β-Lactams having the formula

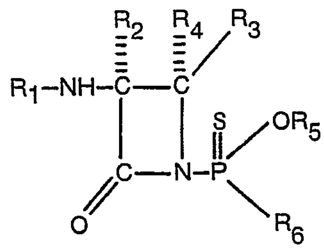

I

,

and salts thereof, have antibacterial activity. As used in formula I, and throughout the specification, the symbols are as defined below:

$R_1$ is acyl;

$R_2$ is hydrogen or methoxy;

$R_3$ and $R_4$ are the same or different and each is hydrogen, akyl, substituted alkyl, or aryl;

$R_5$ is hydrogen, alkyl, substituted alkyl, or aryl; and

$R_6$ is hydroxy, alkoxy, (substituted alkyl)oxy, aryloxy, alkyl, substituted alkyl, aryl, amino, substituted amino, alkylthio or arylthio.

Listed below are definitions of various terms used to describe the β-lactams of this invention. These definitions apply to the terms as they are used throughout the specification (unless they are otherwise limited in specific instances) either individually or as part of a larger group.

The terms "alkyl" and "alkoxy" refer to both straight and branched chain groups having 1 to 10 carbon atoms.

The terms "cycloalkyl" and "cycloalkenyl" refer to cycloalkyl and cycloalkenyl groups having 3, 4, 5, 6 or 7 carbon atoms.

The term "substituted alkyl" refers to alkyl groups substituted with one, or more, azido, amino($-NH_2$), halogen, hydroxy, carboxy, cyano, alkoxycarbonyl, aminocarbonyl, alkoxy, aryloxy, mercapto, alkylthio, arylthio, alkylsulfinyl, alkylsulfonyl groups, arylsulfinyl or arylsulfonyl groups.

The term "alkenyl" refers to both straight and branched chain groups having 2 to 10 carbon atoms.

The term "halogen" refers to fluorine, chlorine, bromine and iodine.

The term "protected carboxyl" refers to a carboxyl group which has been esterified with a conventional acid protecting group. These groups are well known in the art; see, for example, United States patent 4,144, 333, issued March 13, 1979. The preferred protected carboxyl groups are benzyl, benzhydryl, *t*-butyl and *p*-nitrobenzyl esters.

The term "aryl" refers to phenyl or phenyl substituted with 1, 2 or 3 amino, halogen, hydroxyl, trifluoromethyl, alkyl (of 1 to 4 carbon atoms), alkoxy (of 1 to 4 carbon atoms), or carboxyl groups.

The term "substituted amino" refers to a group having the formula $-NY_1Y_2$ wherein $Y_1$ is hydrogen, alkyl, aryl, or arylalkyl, and $Y_2$ is alkyl, aryl, arylalkyl, hydroxy, cyano, alkoxy, phenylalkoxy, or amino.

The term "acyl" refers to acyl groups which have been used in the past to acylate β-lactam antibiotics including 6-aminopenicillanic acid and derivatives and 7-aminocephalosporanic acid and derivatives; see for example, *Cephalosporins and Penicillins,* edited by Flynn, Academic Press (1972), German Offenlegungsschrift 2,716,677, published October 10, 1978, Belgian patent 867,994, published December 11, 1978, United States patent 4,152,432, issued May 1, 1979, United States patent 3,971,778, issued July 27, 1976, United States patent 4,172,199, issued October 23, 1979, and British patent 1,348,894, published March 27, 1974. The following list of acyl groups is presented to further exemplify the term "acyl". Examplary acyl groups are:

(a) Aliphatic groups having the formula

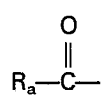

wherein $R_a$ is alkyl; cycloalkyl; alkoxy; alkenyl; cycloalkenyl; cyclohexadienyl; or alkyl or alkenyl substituted with one or more halogen, cyano, nitro, amino, mercapto, alkylthio, or cyanomethylthio groups.

(b) Carbocyclic aromatic groups having the formula

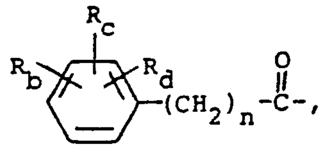 , 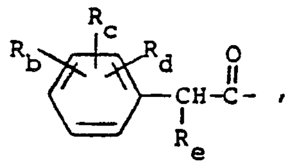 , 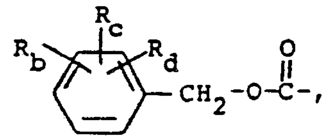 ,

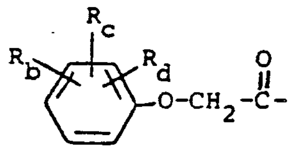 , 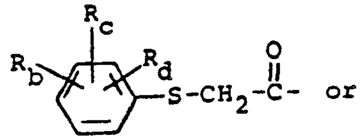 or 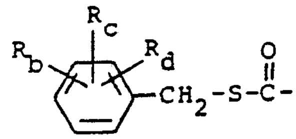

wherein n is 0, 1, 2 or 3; $R_b$, $R_c$, and $R_d$ each is independently hydrogen, halogen, hydroxyl, nitro, amino, cyano, trifluoromethyl, alkyl of 1 to 4 carbon atoms, alkoxy of 1 to 4 carbon atoms or aminomethyl; and $R_e$ is amino, hydroxyl, a carboxyl salt, protected carboxyl, formyloxy, a sulfo salt, a sulfoamino salt, azido, halogen, hydrazino, alkylhydrazino, phenylhydrazino, or [(alkylthio)thiocarbonyl]thio.

Preferred carbocyclic aromatic acyl groups include those having the formula

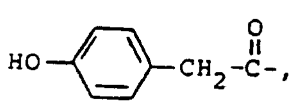 , 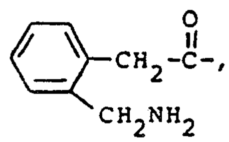 , 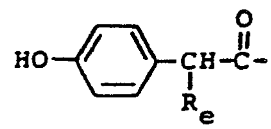

($R_e$ is preferably a carboxyl salt or sulfo salt) and

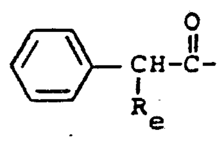

($R_e$ is preferably a carboxyl salt or sulfo salt).

(C) Heteroatomatic groups having the formula

$$R_f{-}(CH_2)_n{-}\overset{\overset{O}{\|}}{C}{-}\ ,\ R_f{-}\underset{\underset{R_e}{|}}{CH}{-}\overset{\overset{O}{\|}}{C}{-}\ ,\ R_f{-}O{-}CH_2{-}\overset{\overset{O}{\|}}{C}{-}\ ,\ R_f{-}S{-}CH_2{-}\overset{\overset{O}{\|}}{C}{-}\ ,\ R_f{-}\overset{\overset{O}{\|}}{C}{-}\overset{\overset{O}{\|}}{C}{-}\ ,$$

wherein n is 0, 1, 2 or 3; $R_e$ is as defined above; and $R_f$ is a substituted or unsubstituted 5-, 6- or 7-membered heterocyclic ring containing 1, 2, 3 or 4 (preferably 1 or 2) nitrogen, oxygen and sulfur atoms. Exemplary heterocyclic rings are thienyl, furyl, pyrrolyl, pyridinyl, pyrazoyl, pyrazinyl, thiazolyl, pyrimidinyl, thiadiazolyl and tetrazolyl. Examplary substituents are substituents are halogen, hydroxyl, nitro, amino, cyano, trifluoromethyl, alkyl of 1 to 4 carbon atoms, alkoxy of 1 to 4 carbon atoms, or

$$HOOC{-}\underset{\underset{NH_2}{|}}{CH}{-}CH_2{-}O{-}\overset{\overset{O}{\|}}{C}{-}NH\ .$$

Preferred heteroaromatic acyl groups include those groups of the above formulas wherein $R_f$ is 2-amino-4-thiazolyl, 2-amino-5-halo-4-thiazolyl, 4-aminopyrimidin-2-yl, 5-amino-1,2,4-thiadiazol-3-yl, 2-thienyl, 2-furanyl, or 5-aminopyridin-2-yl.

(d) [[(4-Substituted-2,3-dioxo-1-piperazinyl)carbonyl]amino]arylacetyl groups having the formula

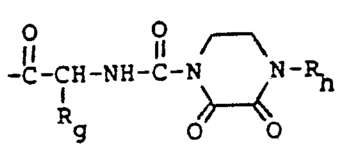

wherein $R_g$ is an aromatic group (including carbocyclic aromatics such as those of the formula

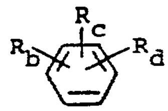

and heteroaromatics as included within the definition of $R_f$); and $R_h$ is alkyl, substituted alkyl (wherein the alkyl group is substituted with one or more halogen, cyano, nitro, amino or mercapto groups), arylmethyleneamino (*i.e.,* —N=CH—$R_g$ wherein $R_g$ is as defined above), arylcarbonylamino (*i.e.,*

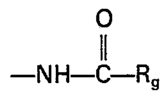

wherein $R_g$ is as defined above) or alkylcarbonylamino.

Preferred [[(4-substituted-2,3-dioxo-1-piperazinyl)carbonyl]amino]arylacetyl groups include those wherein $R_h$ is ethyl, phenylmethyleneamino or 2-furylmethyleneamino.

(e) (Substituted oxyimino)arylacetyl groups having the formula

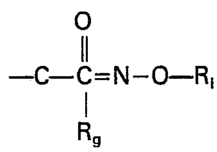

wherein $R_g$ is as defined above and $R_i$ is hydrogen, alkyl, cycloalkyl, alkylaminocarbonyl, arylaminocarbonyl (*i.e.,*

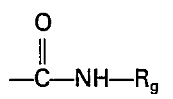

wherein $R_g$ is as defined above) or substituted alkyl (wherein the alkyl group is substituted with 1 or more halogen, cyano, nitro, amino, mercapto, alkylthio, aromatic group (as defined by $R_g$), carboxyl (including salts thereof), amido alkoxycarbonyl, phenylmethoxycarbonyl, diphenylmethoxycarbonyl, hydroxyalkoxyphosphinyl, dihydroxyphosphinyl, hydroxy(phenylmethoxy)phosphinyl, or dialkoxyphosphinyl substituents).

Preferred (substituted oxyimino) arylacetyl groups include those wherein $R_g$ is 2-amino-4-thiazolyl. Also preferred are those groups wherein $R_i$ is methyl, ethyl, carboxymethyl, 1-carboxy-1-methylethyl or 2,2,2-trifluoroethyl.

(f) (Acylamino)arylacetyl groups having the formula

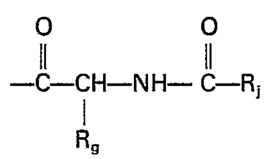

wherein $R_g$ is as defined above and $R_j$ is

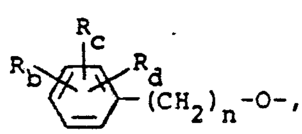

amino, alkylamino, (cyanoalkyl)amino, amido, alkylamido, (cyanoalkyl)amido,

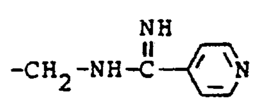 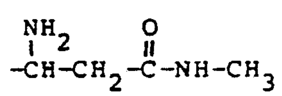

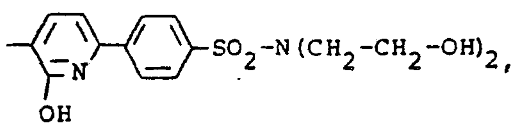, 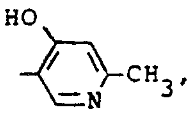,

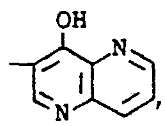, or 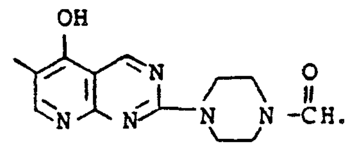.

Preferred (acylamino)arylacetyl groups of the above formula include those groups wherein $R_j$ is amino or amido. Also preferred are those groups wherein $R_g$ is phenyl or 2-thienyl.

(g) [[[3-Substituted-2-oxo-1-imidazolidinyl]carbonyl]amino]arylacetyl groups having the formula

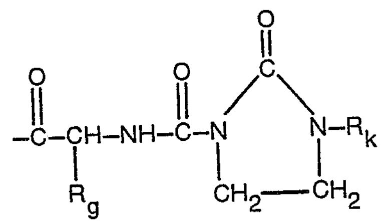

wherein $R_g$ is as defined above and $R_k$ is hydrogen, alkylsulfonyl, arylmethyleneamino (*i.e.*, —N=CH—$R_g$ wherein $R_g$ is as defined above),

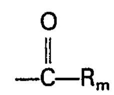

(wherein $R_m$ is hydrogen, alkyl or halogen substituted alkyl), aromatic group (as defined by $R_g$ above), alkyl or substituted alkyl (wherein the alkyl group is substituted with one or more halogen, cyano, nitro, amino or mercapto groups).

Preferred [[3-substituted-2-oxo-1-imidazolidinyl]carbonyl]amino]arylacetyl groups of the above formula include those wherein $R_g$ is phenyl or 2-thienyl. Also preferred are those groups wherein $R_k$ is hydrogen, methylsulfonyl, phenylmethyleneamino or 2-furylmethyleneamino.

The compounds of this invention form basic salts with various inorganic and organic bases which are also within the scope of this invention. Such salts include ammonium salts, alkali metal salts, alkaline earth metal salts, salts with organic bases, *e.g.,* dicyclohexylamine, benzathine, N-methyl-D-glucamine, hydrabamine. The pharmaceutically acceptable salts are preferred, although other salts are also useful, *e.g.,* in isolating or purifying the product.

Some of the products of this invention may be crystallized or recrystallized from solvents containing water. In these cases water of hydration may be formed. This invention contemplates stoichiometric hydrates as well as compounds containing variable amounts of water that may be produced by processes such as lyophilization.

β-Lactams having a

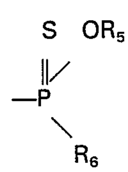

substituent in the 1-position and an amino or acylamino substituent in the 3-position contain at least one chiral center — the carbon atom (in the 3-position of the β-lactam nucleus) to which the amino or acylamino substituent is attached. The invention is directed to those β-lactams which have been described above, wherein the stereochemistry at the chiral center in the 3-position of the β-lactam nucleus is the same as the configuration at the carbon atom in the 6-position of naturally occurring penicillins (*e.g.*, pencillin G) and as the configuration at the carbon atom in the 7-position of naturally occurring cephamycins (*e.g.,* cephamycin C).

Also included within the scope of this invention are racemic mixtures which contain the above-described β-lactams.

The β-lactams of this invention, and salts thereof, have activity against a range of gram-negative and

gram-positive organisms. They can be used as agents to combat bacterial infections (including uinary tract infections and respiratory infections) in mammalian species, such as domesticated animals (*e.g.,* dogs, cats, cows, horses) and humans.

For combating bacterial infections in mammals a compound of this invention can be administered to a mammal in need thereof in an amount of about 1.4 mg/kg/day to about 350 mg/kg/day, preferably about 14 mg/kg/day to about 100 mg/kg/day. All modes of administration which have been used in the past to deliver penicillins and cephalosporins to the site of the infection are also contemplated for use with the β-lactams of this invention. Such methods of administration include oral, intravenous, intramuscular, and as a suppository.

The β-lactam antibiotics of this invention can be prepared from the corresponding azetidine having the formula

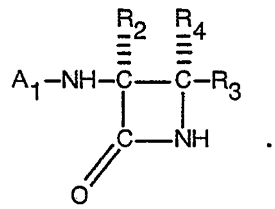

II

In formula II, and throughout the specification, the symbol "$A_1$" refers to an amino protecting group. These groups are well known in the field of β-lactam chemistry and the particular group chosen is not critical. Benzyloxycarbonyl, trityl and *t*-butoxycarbonyl are exemplary protecting groups.

Thiophosphorylation of an azetidine of formula II can be accomplished by first converting the azetidine to a salt having the formula

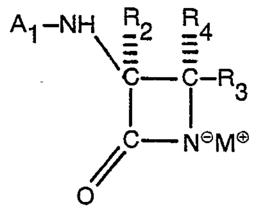

III

($M^{\oplus}$ is a cation) by reaction with a strong base, and then reacting the salt with an activated phosphorous derivative having the formula

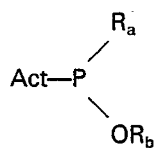

IV

(Act is an activating group, *e.g.,* a halogen atom, $R_b$ is alkyl, sustituted alkyl, or aryl, and $R_a$ is halogen, alkoxy, (substituted alkyl)oxy, aryloxy, alkylthio, alkyl, substituted alkyl, aryl, or disubstituted amino) to yield an azetidine having the formula

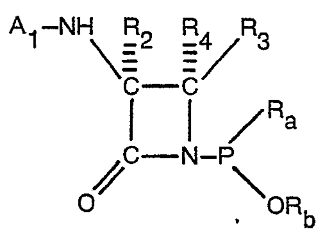

V

Oxidation of an azetidine of formula V with sulfur yields the corresponding azetidine having the formula

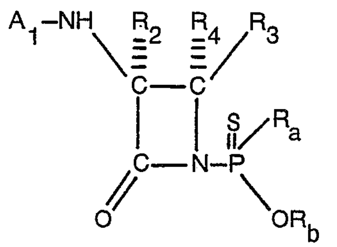 VI

Alternatively, thiophosphorylation of an azetidine of formula III can be accomplished by reaction of the azetidine with a phosphorous derivative having the formula

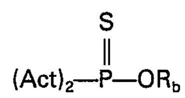 VII

("Act" is preferably chlorine) to yield an azetidine having the formula

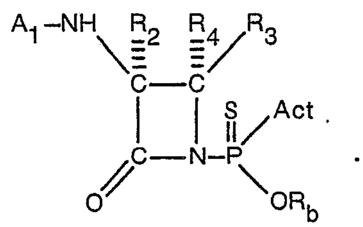 VIII

Reaction of a compound of formula VI wherein $R_a$ is halogen or a compound of formula VIII with ammonia or an amine having the formula

$$HNY_1Y_2$$

yields the corresponding azetidine having the formula

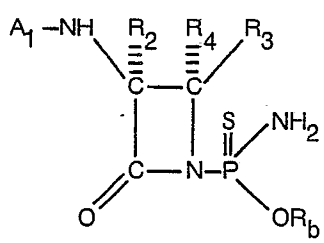

Xa

or

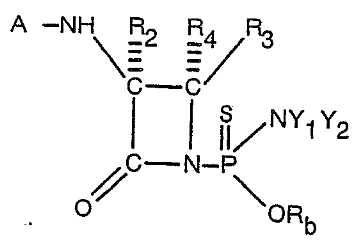

Xb

Reaction of a compound of formula VI wherein $R_a$ is halogen or a compound of formula VIII with water, or an alcohol or thiol having the formula

$$HX—R_c, \qquad XI$$

wherein X is oxygen or sulfur and $R_c$ is alkyl, substituted alkyl or aryl yields the corresponding azetidine having the formula

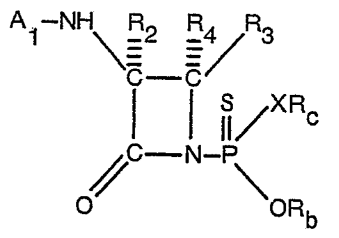

or

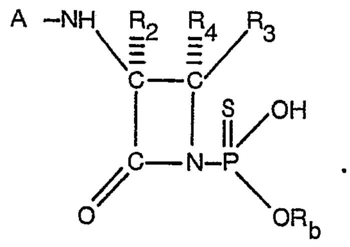

XIIa XIIb

Reaction of a compound having the formula

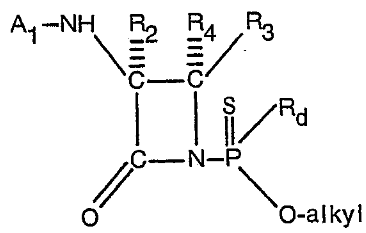

XIII

wherein $R_d$ is alkyl, substituted alkyl, aryl, alkoxy, (substituted alkyl)oxy, aryloxy, alkylthio, (substituted alkyl)thio, or arylthio (see compounds of formulas VI and XII) with a silylating agent (*e.g.,* trimethylsilyl bromide) in the presence of an acid scavenger (*e.g.,* bis-(trimethylsilyl)trifluoroacetamide) followed by treatment with water and an organic or inorganic base yields a salt of the corresponding azetidine having the formula

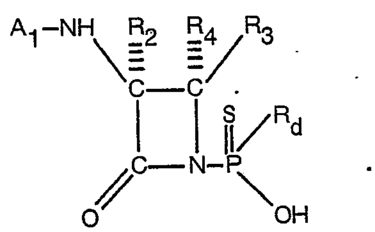

XIV

Alternatively, reaction of an azetidine of formula XIII with thiourea in an organic solvent such as acetonitrile, followed by the appropriate ion transfer method (*e.g.,* ion exchange chromatography), can be used to obtain a salt of the azetidine of formula XIV.

Salts of diacidic compounds having the formula

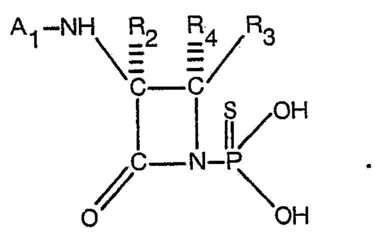

XV

can be formed by treating a dialkyl ester of formula XIII ($R_d$ is alkoxy) with excess trimethylsilyl bromide and bis-(trimethylsilyl)acetamide followed by treatment with an organic or inorganic base.

The following examples are specific embodiments of this invention.

Example 1

[3S(Z)]-2-[[[1-(2-Amino-4-thiazolyl)-2-[[1-(ethoxyhydroxyphosphinothioyl)-2-oxo-3-azetidinyl]amino]-2-oxoethylidene]amino]oxy]-2-methylpropanoic acid, monoethyl ester, dipotassium salt

A) (S)-[1-(Diethoxyphosphinothioyl)-2-oxo-3-azetidinyl]carbamic acid, 1,1-dimethylethoxyester

A solution of (S)-3-[[(1,1-dimethylethoxy)-carbonyl]amino]-2-azetidinone (1.12 g) in 40 ml of dry tetrahydrofuran at −78°C was treated with 4.1 ml of 1.61 N n-butyl lithium. After 30 minutes, 0.86 ml of diethyl chlorophosphite was added in one portion. After stirring for 100 minutes at −75°C, 384 mg of

powdered sulfur was added in one portion at −75°C. The reaction mixture was allowed to warm to room temperature and stirred overnight at 5°C.

The reaction product was extracted three times from monobasic potassium phosphate/sodium chloride/water with ethyl acetate. The combined organic layers were dried with sodium sulfate and the solvent was evaporated. Column chromatography of the residue on silica eluting with 4:1 hexane/ethyl acetate yielded 1.06 g of the title compound. Trituration with petroleum ether yielded a waxy solid, melting point 60—61°C.

B) (S)-[1-(Ethoxyhydroxyphosphinothioyl)-2-oxo-3-azetidinyl]carbamic acid, 1,1-dimethylethoxy ester, potassium salt

To 1.06 g of (S)-[1-(diethoxyphosphinothioyl)-2-oxo-3-azetidinyl]carbamic acid, 1,1-dimethylethoxy ester in 24 ml of acetonitrile was added 1.26 g of thiourea. After refluxing for 15 hours, acetonitrile was removed by boiling at one atmosphere until a small amount of precipitate was evident. After 24 hours at reflux, 0.65 g of thiourea was added and the reaction mixture was refluxed for an additional 15 hours. The volatiles were evaporated under vacuum, and the residue was chromatographed on a Dowex® 50X2—400 ($K^{\oplus}$ form) column (eluting with water) followed by an HP—20® column (eluting with water, 10% acetone—water and 20% acetone—water) to yield 334 mg of the title compound.

C) [3S(Z)]-2-[[[1-(2-Amino-4-thiazolyl)-2-[[1-(ethoxyhydroxyphosphinothioyl)-2-oxo-3-azetidinyl]amino]-2-oxoethylidene]amino]oxy]-2-methylpropanoic acid, benzhydryl ester, potassium salt

Diisopropylethylamine (0.116 ml) was added to (Z)-2-amino-α-[[2-(diphenylmethoxy)-1,1-dimethyl-2-oxoethoxy]imino]-4-thiazoleacetic acid (264 mg) in 2 ml of acetonitrile at room temperature. The mixture was cooled to −20°C, diphenyl chlorophosphate (0.124 ml) was added, and the resulting mixture was stirred for 30 minutes to yield a mixed anhydride.

(S)-[1-(Ethoxyhydroxyphosphinothioyl]-2-oxo-3-azetidinyl]carbamic acid, 1,1-dimethylethoxy ester, potassium salt (208 mg) was dissolved in 0.3 ml of anisole and cooled to 0°C. Trifluoroacetic acid (3 ml) was added, and the resulting mixture was stirred at 0°C for 30 minutes. The volatiles were evaporated, and the residue was triturated with petroleum ether and anhydrous ether to yield the trifluoroacetic acid salt of (S)-(3-amino-2-oxo-1-azetidinyl)thiophosphonic acid, ethyl ester. After evacuation for 45 minutes, the residue was dissolved in 2 ml of acetonitrile, and upon cooling to 0°C, 0.5 ml of diisopropylethylamine was added. The reaction mixture containing the mixed anhydride was added to the azetidinone.

After stirring at 5°C overnight, the volatiles were removed under vacuum. The residue was purified by column chromatography with 20% acetone-water on Dowex® 50X2—400 ($K^{\oplus}$ form) followed by chromatography on HP—20AG®* resin (eluting with 40% acetone-water) to yield 230 mg of the title compound.

D) [3S(Z)]-2-[[[1-(2-Amino-4-thiazolyl)-2-[[1-(ethoxyhydroxyphosphinothioyl)-2-oxo-3-azetidinyl]amino]-2-oxoethylidene]amino]oxy]-2-methylpropanoic acid, monoethyl ester, dipotassium salt.

[3S(Z)] - 2 - [[[1 - (2 - Amino - 4 - thiazolyl) - 2 - [[1 - (ethoxyhydroxyphosphinothioyl) - 2 - oxo - 3 - azetidinyl]amino - 2 - oxoethylidene]amino]oxy] - 2 - methylpropanoid acid, benzhydryl ester, potassium salt (334 mg) was dissolved in 2.4 ml of anisole and cooled to 0°C. Trifluoroacetic acid (4.8 ml) was added, and the resulting mixture was stirred at 5°C for 2 hours. The volatiles were evaporated, and the residue was triturated with petroleum ether and anhydrous ether. The residue was dissolved in aqueous potassium bicarbonate and purified by chromatography on HP—20AG® resin (eluting with water) to yield 173 mg of the title compound, melting point 200°C, *dec.*

Example 2

[3S(Z)]-2-[[[1-(2-Amino-4-thiazolyl)-2-[[1-(dihydroxyphosphinothioyl)-2-oxo-3-azetidinyl]-amino]-2-oxoethylidene]amino]oxy]-2-methylpropanoic acid, potassium salt

A) (S)-[1-(Dihydroxyphosphinothioyl)-2-oxo-3-azetidinyl]carbamic acid, 1,1-dimethylethoxy ester, dipotassium salt

To 5.07 g of (S)-[1-(diethoxyphosphinothioyl)-2-oxo-3-azetidinyl]carbamic acid, 1,1-dimethylethoxy ester (see example 1A) in 20 ml of methylene chloride is added 11.3 ml of bis(trimethylsilyl)acetamide, and the solution is stirred at room temperature for 15 minutes. Trimethylsilyl bromide (6.02 ml) is then added, and the reaction is stirred at ambient temperature for 1.5 hours. The volatiles are evaporated, the residue dissolved in toluene, and the volatiles removed again. The resulting oil is dissolved in 10 ml of tetrahydrofuran, and 2.83 g of aniline is added followed by 5 ml of ethanol. The resulting solution is stored overnight at −15°C to give (S)-[1-(dihydroxyphosphinothioyl)-2-oxo-3-azetidinyl]carbamic acid, 1,1-dimethylethoxy ester, monoanilinium salt. The anilinium salt is dissolved in water and neutralized with potassium bicarbonate. The neutral solution is then applied to a Dowex® 50X2—400 column ($K^{\oplus}$ form, eluting with water) to give the title compound.

---

* HP—20AG® resin is a macroporous styrene-divinyl-benzene copolymer manufactured by Mitsubishi Chemical Industries.

B) [3S(Z)]-2-[[[1-(2-Amino-4-thiazolyl)-2-[[1-(dihydroxyphosphinothioyl)-2-oxo-3-azetidinyl]amino]-2-oxoethylidene]amino]oxy]-2-methylpropanoic acid, benzhydryl ester, dipotassium salt

Diisopropylethylamine (0.116 ml) is added to (Z)-2-amino-α-[[2-diphenylmethoxy)-1,1-dimethyl-2-oxoethoxy]imino]-4-thiazoleacetic acid (264 mg) in 2 ml of acetonitrile at room temperature. The mixture is cooled to −20°C, diphenyl chlorophosphate (0.124 ml) is added, and the resulting mixture is stirred for 30 minutes to yield a mixed anhydride.

(S)-[1-(Dihydroxyphosphinothioyl)-2-oxo-3-azetidinyl]carbamic acid, 1,1-dimethylethoxy ester, dipotassium salt (215 mg) is dissolved in 0.3 ml of anisole and cooled to 0°C. Trifluoroacetic acid (3 ml) is added, and the resulting mixture is stirred at 0°C for 30 minutes. The volatiles are evaporated, and the residue is triturated with petroleum ether and anhydrous ether to yield the trifluoroacetic acid salt of (S)-(3-amino-2-oxo-1-azetidinyl)thiophosphonic acid. After evacuation for 45 minutes, the residue is dissolved in 2 ml of water, cooled to 5°C, and neutralized with potassium bicarbonate. The reaction mixture containing the mixed anhydride is then added to the azetidinone.

After stirring at 5°C overnight, the volatiles are removed under vacuum. The residue is purified by column chromatography on Dowex® 50X2—400 (K⊕ form) followed by chromatography on HP—20AG® resin to give the title compound.

C) [3S(Z)]-2-[[[1-(2)-Amino-4-thiazolyl)-2-[[1-(dihydroxyphosphinothioyl)-2-oxo-3-azetidinyl]-amino]-2-oxoethylidene]amino]oxy]-2-methylpropanoic acid, potassium salt.

[3S(Z)] - 2 - [[[1 - (2 - Amino - 4 - thiazolyl) - 2 - [[1 - (dihydroxyphosphinothioyl) - 2 - oxo - 3 - azetidinyl]amino] - 2 - oxoethylidene]amino]oxy] - 2 - methylpropanoic acid, benzhydryl ester, dipotassium salt (300 mg) is dissolved in 2.4 ml of anisole and cooled to 0°C. Trifluoroacetic acid (4.8 ml) is added, and the resulting mixture is stirred at 5°C for 2 hours. The volatiles are evaporated, and the residue is triturated with petroleum ether and anhydrous ether. The residue is dissolved in water, and the pH is adjusted to 2.5 with potassium bicarbonate. This solution is subjected to chromatography on HP—20AG® resin to yield the title compound.

Example 3

[3S(Z)]-2-[[[1-(2-Amino-4-thiazolyl)-2-[[1-[(hydroxy)(n-propylamino)phosphinothioyl]-2-oxo-3-azetidinyl]amino]-2-oxoethylidene]amino]oxy]-2-methylpropanoic acid, dipotassium salt

A) (S)-[1-[(Methoxy)(n-propylamino)phosphinothioyl]-2-oxo-3-azetidinyl]carbamic acid, 1,1-dimethylethoxy ester

To 4.1 g of (S)-[[(1,1-dimethylethoxy)carbonyl]amino-2-azetidinone dissolved in dry tetrahydrofuran (140 ml) and cooled to −78°C is added 14.2 ml of 1.56 M n-butyl lithium. The mixture is stirred for 30 minutes at −78°C. O-Methylphosphorodichloroidothioate (3.63 ml) is added and the mixture is stirred for 2 hours at −78°C. n-Propylamine (2.60 g) is then added, and the reaction is stirred for an additional 2 hours at −78°C. The reaction is treated with 0.5 M phosphate buffer (pH 5.5, 100 ml), allowed to warm to 5°C, and extracted with ethyl acetate (three 125 ml portions). The extracts are combined, dried over sodium sulfate, and the solvent removed *in vacuo*. The residue is chromatographed on silica to give the title compound.

B) (S)-[1-[(Hydroxy)(n-propylamino)phosphinothioyl]-2-oxo-3-azetidinyl]carbamic acid, 1,1-dimethylethoxy ester, potassium salt

(S)-1-[(Methoxy)(n-propylamino)phosphinothioyl]-2-oxo-3-azetidinyl]carbamic acid, 1,1-dimethylethoxy ester (2.82 g) is dissolved in 30 ml of methylene chloride and cooled to 0°C. Bis(trimethylsilyl)acetamide (6.88 g) is added and the mixture is stirred at 0°C for 0.5 hour. Trimethylsilyl bromide (3.9 g) is added and the mixture stirred for 3 hours at 5°C. The volatiles are removed *in vacuo*, the residue is dissolved in toluene, and the volatiles are removed again. The residue is dissolved in dry tetrahydrofuran (50 ml), aniline (1.57 g) in ethanol (15 ml) is added, and the mixture is stored at −15°C overnight to give the desired monoanilinium salt. The anilinium salt is dissolved in water and subjected to a Dowex® 50X2—400 column (K⊕ form) to give the title compound upon lyophilization.

C) [3S(Z)]-2-[[[1-(2-Amino-4-thiazolyl)-2-[[1-[(hydroxy)(n-propylamino)phosphinothioyl]-2-oxo-3-azetidinyl]amino]-2-oxoethylidene]amino]oxy]-2-methylpropanoic acid, benzhydryl ester, potassium salt

Diisopropylethylamine (0.116 ml) is added to (Z)-2-amino-α-[[2-(diphenylmethoxy)-1,1-dimethyl-2-oxoethoxy]imino]-4-thiazoleacetic acid (264 mg) in 2 ml of acetonitrile at room temperature. The mixture is cooled to −20°C, diphenyl chlorophosphate (0.124 ml) is added, and the resulting mixture is stirred for 30 minutes to yield a mixed anhydride.

(S)-[1-[(Hydroxy)(n-propylamino)phosphinothioyl]-2-oxo-3-azetidinyl]carbamic acid, 1,1-dimethylethoxy ester, potassium salt (216 mg) is dissolved in 0.3 ml of anisole and cooled to 0°C. Trifluoroacetic acid (3 ml) is added, and the resulting mixture is stirred at 0°C for 30 minutes. The volatiles are evaporated, and the residue is triturated with petroleum ether and anhydrous ether to yield the trifluoroacetic acid salt. After evacuation for 45 minutes, the residue is dissolved in 2 ml of acetonitrile, and upon cooling to 0°C, 0.5 ml of diisopropylethylamine is added. The reaction mixture containing the mixed anhydride is added to the azetidinone.

After stirring at 5°C overnight, the volatiles are removed under vacuum. The residue was purified by

column chromatography on Dowex® 50X2—400 (K⊕ form) followed by chromatography on HP—20AG® resin to give the title compound.

D) [3S(Z)]-2-[[[1-(2-Amino-4-thiazolyl)-2-[[1-[(hydroxy)(n-propylamino)phosphinothioyl]-2-oxo-3-azetidinyl]amino-2-oxoethylidene]amino]oxy]-2-methylpropanoic acid, dipotassium salt

[3S(Z)] - 2 - [[[1 - (2 - Amino - 4 - thiazolyl) - 2 - [[1 - [(hydroxy)(n - propylamino)phosphinothioyl] - 2 - oxo - 3 - azetidinyl]amino] - 2 - oxoethylidene]amino]oxy - 2 - methylpropanoic acid, benzhydryl ester, potassium salt (185 mg) is dissolved in 1.5 ml of anisole and cooled to 0°C. Trifluoroacetic acid (3.0 ml) is added, and the resulting mixture is stirred at 5°C for 2 hours, the volatiles are evaporated, and the residue is triturated with petroleum ether and anhydrous ether. The residue is dissolved in water, and the solution is neutralized with potassium bicarbonate. The solution is subjected to chromatography on HP—20AG® resin to yield the title compound.

Exemplary of additional compounds which can be prepared utilizing the procedures described above are the following:

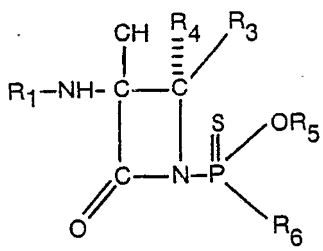

,

| | $R_1$ | $R_3$ | $R_4$ | $R_5$ | $R_6$ |
|---|---|---|---|---|---|
| i | 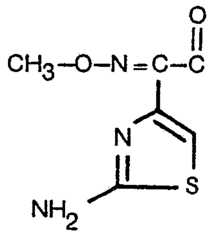 | H | H | $CH_3$ | $O^{\ominus} K^{\oplus}$ |
| ii | 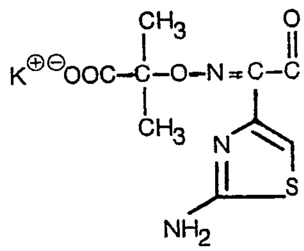 | H | H | $CH_3$ | $O^{\ominus} K^{\oplus}$ |
| iii | 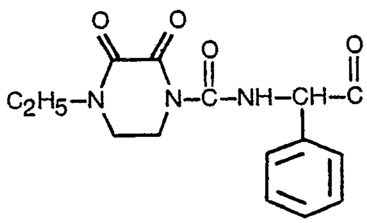 | H | H | $CH_3$ | $O^{\ominus} K^{\oplus}$ |
| iv | 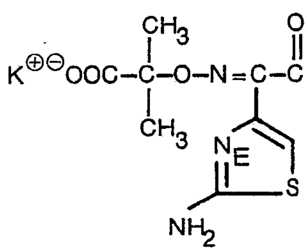 | H | $CH_3$ | $CH_3$ | $O^{\ominus} K^{\oplus}$ |
| v | 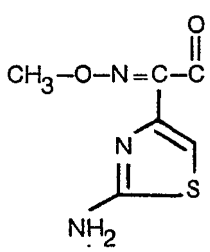 | H | $CH_3$ | $CH_3$ | $O^{\ominus} K^{\oplus}$ |
| vi | 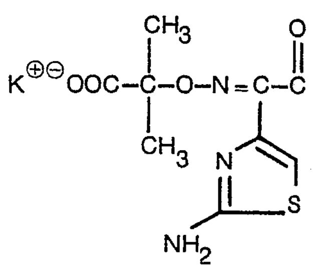 | $CH_3$ | H | $CH_3$ | $O^{\ominus} K^{\oplus}$ |

| | $R_1$ | $R_3$ | $R_4$ | $R_5$ | $R_6$ |
|---|---|---|---|---|---|
| vii | 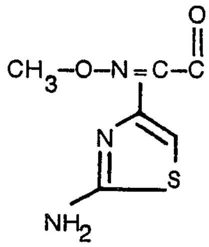 | $CH_3$ | H | $CH_3$ | $O^{\ominus}\ K^{\oplus}$ |
| viii | 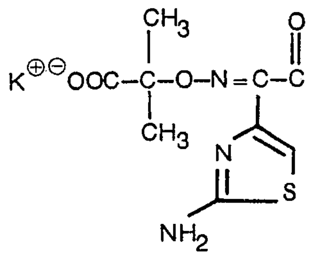 | H | $CH_3$ | $CH_3CH_2$ | $O^{\ominus}\ K^{\oplus}$ |
| ix | 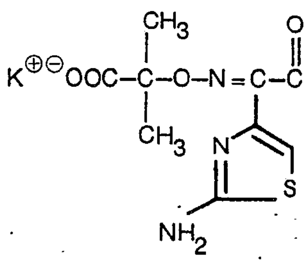 | $CH_3$ | H | $CH_3CH_2$ | $O^{\ominus}\ K^{\oplus}$ |
| x | 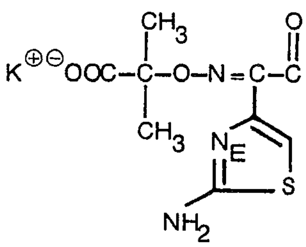 | H | H | $K^{\oplus}$ | $O^{\ominus}\ K^{\oplus}$ |
| xi | 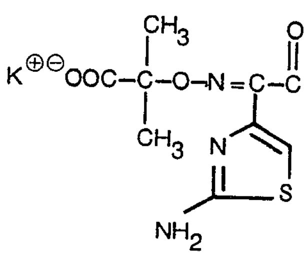 | H | H | $K^{\oplus}$ | $O^{\ominus}\ K^{\oplus}$ |

## Claims

1. A β-lactam having the formula

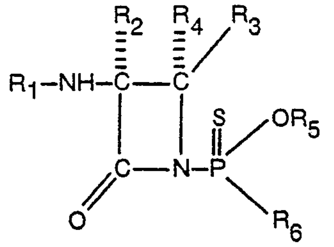

,

or a pharmaceutically acceptable salt thereof,
wherein

$R_1$ is acyl as employed in beta-lactam antibiotics;

$R_2$ is hydrogen or methoxy;

$R_3$ and $R_4$ are the same or different and each is hydrogen, alkyl, substituted alkyl or aryl;

$R_5$ is hydrogen, alkyl, substituted alkyl, or aryl; and

$R_6$ is hydroxy, alkoxy, (substituted alkyl)oxy, aryloxy, alkyl, substituted alkyl, aryl, amino, substituted amino, alkylthio or arylthio,

wherein alkyl and alkoxy are straight or branched chain groups having 1 to 10 carbon atoms; substituted alkyl is an alkyl group as defined above substituted with azido, amino, halogen, hydroxy, carboxy, cyano, alkoxycarbonyl, aminocarbonyl, alkoxy, aryloxy, mercapto, alkylthio, arylthio, alkylsulfinyl, alkylsulfonyl, arylsulfinyl or arylsulfonyl groups; halogen is fluorine, chlorine, bromine or iodine; aryl is phenyl or phenyl substituted with 1, 2 or 3 amino, halogen, hydroxyl, trifluoromethyl, alkyl (of 1 to 4 carbon atoms), alkoxy (of 1 to 4 carbon atoms), or carboxyl groups; and substituted amino is a group having the formula $—NY_1Y_2$ wherein $Y_1$ is hydrogen, alkyl, aryl, or arylalkyl, and $Y_2$ is alkyl, aryl, arylalkyl, hydroxy, cyano, alkoxy, phenylalkoxy, or amino.

2. A β-lactam in accordance with claim 1 wherein $R_2$ is hydrogen.

3. A β-lactam in accordance with claim 2 wherein $R_3$ and $R_4$ are the same or different and each is hydrogen or alkyl.

4. A β-lactam in accordance with claim 2 wherein $R_3$ and $R_4$ are each hydrogen.

5. A β-lactam in accordance with claim 2 wherein $R_5$ is hydrogen.

6. A compound in accordance with claim 2 wherein $R_5$ is hydrogen and $R_6$ is alkoxy.

7. A compound in accordance with claim 2 wherein $R_1$ is (Z)-2-amino-α-[(1-carboxy-1-methylethoxy)-imino]-4-thiazoleacetyl.

8. The compound in accordance with claim 1, [3S(Z)] - 2 - [[[1 - (2 - amino - 4 - thiazolyl) - 2 - [[1 - (ethoxyhydroxyphosphinothioyl) - 2 - oxo - 3 - azetidinyl]amino] - 2 - oxoethylidene]amino]oxy] - 2 - methylpropanoic acid, monoethyl ester, dipotassium salt.

## Patentansprüche

1. β-Lactam mit der Formel

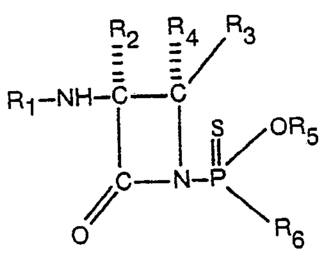

,

oder dessen pharmakologisch verträgliche Salze, in dem

$R_1$ eine in der Chemie der β-Lactam-Antibiotika verwendete Acylgruppe bedeutet,

$R_2$ ein Wasserstoffatom oder eine Methoxygruppe bedeutet,

$R_3$ und $R_4$ gleich oder verschieden sind und jedes ein Wasserstoffatom, ein Alkyl- oder substituierter Alkylrest oder ein Arylrest sein kann,

$R_5$ ein Wasserstoffatom, ein Alkyl- oder substituierter Alkyl-rest oder ein Arylrest ist, und

$R_6$ eine Hydroxylgruppe, ein Alkoxy- oder substituierter Alkoxyrest, ein Aryloxyrest, ein Alkyl- oder substituierter Alkylrest, ein Arylrest, eine Amino- oder substituierte Aminogruppe, ein Alkylthio- oder Arylthiorest ist; hierbei sind Alkyl- und Alkoxyreste geradkettige oder verzweigte Reste mit 1 bis 10 Kohlenstoffatomen,

substituierte Alkylreste sind Alkylreste wie vorstehend definiert, welche Azido- und Aminogruppen, Halogenatome, Hydroxyl-, Carboxyl- und Cyanogruppen, Alkoxycarbonyl-, Aminocarbonyl-, Alkoxy- oder Aryloxyreste, Mercaptogruppen, Alkylthio-, Arylthio-, Alkylsulfinyl-, Alkylsulfonyl-, Arylsulfinyl- oder Arylsulfonylreste tragen, Halogen, bedeutet ein Fluor-, Chlor-, Brom- oder Jodatom, der Arylrest ist eine Phenylgruppe oder eine Phenylgruppe, welche 1, 2 oder 3 Aminogruppen, Halogenatome, Hydroxyl- oder Trifluormethylgruppen, Alkylreste mit 1 bis 4 Kohlenstoffatomen, Alkoxyreste mit 1 bis 4 Kohlenstoffatomen oder Carboxylgruppen trägt, die substituierte Aminogruppe ist ein Rest mit der Formel $—NY_1Y_2$, in der $Y_1$ ein Wasserstoffatom, ein Alkyl-, Aryl- oder Arylalkylrest, und $Y_2$ ein Alkyl-, Aryl- oder Arylalkylrest, eine Hydroxyl- oder Cyanogruppe, ein Alkoxy- oder Phenylalkoxyrest oder eine Aminogruppe ist.

2. β-Lactam nach Anspruch 1, in dem $R_2$ ein Wasserstoffatom ist.

3. β-Lactam nach Anspruch 2, in dem $R_3$ und $R_4$ gleich oder verschieden sind und jedes ein Wasserstoffatom oder ein Alkylrest sein kann.

4. β-Lactam nach Anspruch 2, in dem $R_3$ und $R_4$ jeweils Wasserstoffatome sind.

5. β-Lactam nach Anspruch 2, in dem $R_5$ ein Wasserstoffatom ist.

6. Eine Verbindung nach Anspruch 2, in der $R_5$ ein Wasserstoffatom und $R_6$ ein Alkoxyrest ist.

7. Eine Verbindung nach Anspruch 2, in der $R_1$ der Rest (Z)-2-Amino-α-[(1-carboxy-1-methyläthoxy)-imino]-4-thiazolylacetyl ist.

8. Eine Verbindung nach Anspruch 1, nämlich das Dikaliumsalz des [3S(Z)] - 2 - [[[1 - (2 - Amino - 4 - thiazolyl) - 2 - [[1(äthoxyhydroxyphosphinothioyl) - 2 - oxo - 3 - azetidinyl] - amino] - 2 - oxoäthyliden] - amino] - oxy] - 2 - methylpropionsäuremonoäthylesters.

**Revendications**

1. β-lactame ayant pour formule

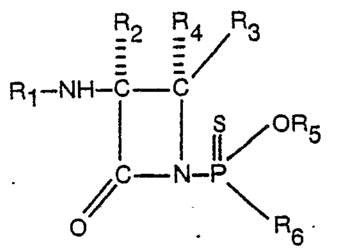

,

éventuellement sous la forme d'un sel pharmaceutiquement acceptable,
formule dans laquelle $R_1$ est un radical acyle tel qu'il est employé dans les antibiotiques de la série des bêta-lactames;

$R_2$ est un atome d'hydrogène ou un groupement méthoxy;

$R_3$ et $R_4$ sont identiques ou différents et sont chacun un atome d'hydrogène, un radical alkyle éventuellement substitué, ou un radical aryle;

$R_5$ est un atome d'hydrogène, un radical alkyle éventuellement substitué, ou un radical aryle; et

$R_6$ est un groupement hydroxy, alcoxy, (alkyl substitué)oxy, aryloxy, alkyle éventuellement substitué, aryle, amine éventuellement substitué, alkylthio ou arylthio,

les groupements alkyle et alcoxy étant des groupements à chaîne droite ou ramifiée de 1 à 10 atomes de carbone; le groupement alkyle substitué étant un groupement alkyle tel qu'il est défini ci-dessus, substitué par des atomes ou des groupements azide, amine, halogène, hydroxy, carboxy, cyano, alcoxycarbonyle, aminocarbonyle, alcoxy, aryloxy, mercapto, alkylthio, arylthio, alkylsulfinyle, alkylsulfonyle, arylsulfinyle ou arylsulfonyle; les atomes d'halogène étant des atomes de fluor, de chlore, de brome ou d'iode; le groupement aryle étant un groupement phényle éventuellement substitué par 1, 2 ou 3 atomes ou groupements amine, halogène, hydroxyle, trifluorométhyle, alkyle (de 1 à 4 atomes de carbone), alcoxy (de 1 à 4 atomes de carbone), ou carboxyle; et le groupement amine substitué étant un groupement de formule $—NY_1Y_2$ dans laquelle $Y_1$ est un atome d'hydrogène ou un radical alkyle, aryle ou arylalkyle et $Y_2$ est un radical alkyle, aryle, arylalkyle, hydroxy, cyano, alcoxy, phénylalcoxy ou amine.

2. β-lactame selon la revendication 1, dans la formule duquel $R_2$ est un atome d'hydrogène.

3. β-lactame selon la revendication 2, dans la formule duquel $R_3$ et $R_4$ sont identiques ou différents et sont chacun un atome d'hydrogène ou un radical alkyle.

4. β-lactame selon la revendication 2, dans la formule duquel $R_3$ et $R_4$ sont chacun un atome d'hydrogène.

5. β-lactame selon la revendication 2, dans la formule duquel $R_5$ est un atome d'hydrogène.

6. Composé selon la revendication 2, dans la formule duquel $R_5$ est un atome d'hydrogène et $R_6$ est un radical alcoxy.

7. Composé selon la revendication 2, dans la formule duquel $R_1$ est un radical (Z)-2-amino-α-[(1-carboxy-1-méthyléthoxy)imino]-4-thiazoleacétyle.

8. Composé selon la revendication 1, qui est le sel dipotassique de l'ester monoéthylique de l'acide [3S(Z)] - 2 - [[[1 - (2 - amino - 4 - thiazolyl) - 2 - [[1 - (éthoxyhydroxyphosphinothioyl) - 2 - oxo - 3 - azétidinyl]amino] - 2 - oxoéthylidène]amino]oxy - 2 - méthylpropanoïque.